(19) Europäisches Patentamt
European Patent Office
Office européen des brevets

(11) Veröffentlichungsnummer: **0 137 793**
**B1**

# (12) EUROPÄISCHE PATENTSCHRIFT

(45) Veröffentlichungstag der Patentschrift: **08.10.86**

(51) Int. Cl.⁴: **C 07 C 49/21**, C 07 C 47/225, C 07 C 33/05, C 11 B 9/00, A 61 K 7/46

(21) Anmeldenummer: **84900617.6**

(22) Anmeldetag: **03.02.84**

(86) Internationale Anmeldenummer: **PCT/EP 84/00028**

(87) Internationale Veröffentlichungsnummer: **WO 84/03086 (16.08.84** Gazette **84/20)**

## (54) 2,6-EXO-KONFIGURIERTE TRICYCLO (5.2.1.0(2,6)) DECAN-DERIVATE MIT FUNKTIONALISIERTER SEITENKETTE AN C-8/C-9 UND DEREN VERWENDUNG ALS RIECHSTOFFE.

(30) Priorität: **05.02.83 DE 3303893**

(43) Veröffentlichungstag der Anmeldung: **24.04.85 Patentblatt 85/17**

(45) Bekanntmachung des Hinweises auf die Patenterteilung: **08.10.86 Patentblatt 86/41**

(84) Benannte Vertragsstaaten: **CH DE FR GB LI NL**

(56) Entgegenhaltungen:
**FR - A - 1 416 209**
**GB - A - 2 020 277**
**GB - A - 2 020 656**

**Die Akte enthält technische Angaben, die nach dem Eingang der Anmeldung eingereicht wurden und die nicht in dieser Patentschrift enthalten sind.**

(73) Patentinhaber: **Dragoco Gerberding & Co. GmbH, Dragocostrasse 1, D-3450 Holzminden (DE)**

(72) Erfinder: **BRUNKE, Ernst-Joachim, Holbeinstrasse 6, D-3450 Holzminden (DE)**

(74) Vertreter: **Deufel, Paul, Dr. et al, Patentanwälte Müller-Boré, Deufel, Schön, Hertel, Lewald, Otto Isartorplatz 6 Postfach 26 02 47, D-8000 München 26 (DE)**

**EP 0 137 793 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Dicyclopentadien (1) ist unter anderem ein wichtiges Ausgangsmaterial für die Herstellung von Riechstoffen (Übersicht in: H. Aebi, E. Baumgartner, H.P. Fiedler und G. Ohloff, „Kosmetika, Riechstoffe und Lebensmittelzusatzstoffe", G. Thieme Verlag, Stuttgart 1978, S. 55-57).

Unter den bekannten Riechstoffen mit Tricyclo-[5.2.1.$0^{2,6}$]decan-Grundgerüst befinden sich nur wenige mit funktionalisierter Seitenkette. In der DE-AS 1 218 643 (8.6.1966) werden die im Ringsystem gesättigten Tricyclodecan-

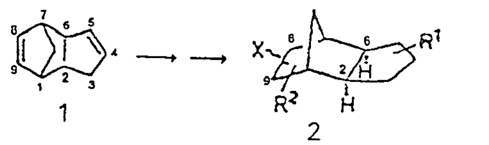

1 2

$R^1, R^2$ = H,$CH_3$
X = -O-CH-COOR
(R=H,$C_1$-$C_4$-Alkyl)
-CHO
-CH=CH-CO-R'
(R'=$C_1$-$C_5$-Alkyl)
-$CH_2$-$CH_2$-CO-R'

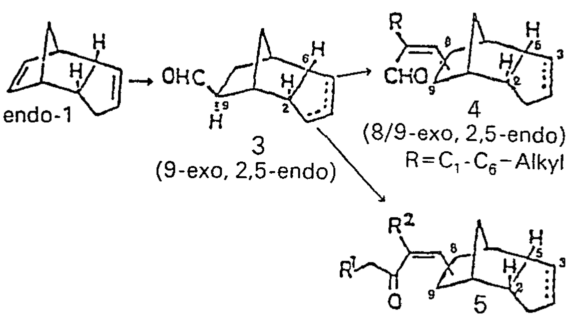

endo-1

3
(9-exo, 2,5-endo)

4
(8/9-exo, 2,5-endo)
R=$C_1$-$C_6$-Alkyl

5
(8/9-exo, 2,6-endo)
$R^1$,$R^2$=H,$C_1$-$C_5$-Alkyl, $C_1$-$C_5$-Alkenyl

Derivate 2 als Riechstoffe beansprucht, bei denen der Rest X eine Glycidat-, Formyl- oder geradkettige 3'-Oxo-alkylgruppe bedeutet. Die Ringverknüpfungen an C-2 und C-6 sollen *exo*-konfiguriert sein; über die Stereochemie der Seitenketten-Verknüpfung an C-8 oder C-9 wird nichts ausgesagt. Der Aldehyd der Formel 2 besitzt einen frischen Geruch mit erdigen Noten, und die ungesättigten Ketone der Formel 2 riechen trocken-holzig mit Wein-Note (R = Me) bzw. trocken-holzig mit Iris-Note (R = Et).

Durch Hydroformylierung des bei Raumtemperatur vorliegenden *endo*-Dicyclopentadiens (endo-1) unter Verwendung von Rhodiumkatalysatoren wurde der Aldehyd 3 mit relativer 9-exo-2,6-endo-Konfiguration erhalten [Y. Fujikura, Y. Inamoto, N. Takaishi und H. Ikeda, Synth. Commun., 6, 199-207 (1976)]. Durch Kondensation dieses Aldehyds 3 mit aliphatischen Aldehyden wurden die ungesättigten Aldehyde 4 mit *exo*-Anordnung der funktionalisierten Seitenkette und *endo*-Verknüpfung an C-2 und C-6 erhalten; die gestrichelte Linie bedeutet eine fakultative Doppelbindung an C-3 [US-PS 4,229,324 (21.10.1980)]. Die Verbindungen der Formel 4 besitzen Geruchsnoten vom Holz-Typ mit Nebennoten von Iriswurzel und Honig und können als Riechstoffe verwendet werden.

Durch Aldolkondensation des 9-exo-2,6-endo-konfigurierten Aldehyds 3 mit aliphatischen Ketonen wurden die ungesättigten Ketone 5 mit *exo*-Anordnung der funktionalisierten Seitenkette und *endo*-Ringverknüpfung an C-2 und C-6 erhalten; die gestrichelte Linie bedeutet eine fakultative Doppelbindung an C-3 [FR-OS 2 425 419 (7.12.1979)]. Die in dieser Schrift beschriebenen Ketone der Formel 5, die als geometrische Isomere (Doppelbindung in der Seitenkette) vorliegen, besitzen holzige Geruchsnoten mit zusätzlichen Geruchsaspekten vom Typ Iriswurzel oder Honig. Die erfindungsgemässen Substanzen der allgemeinen Formel A unterscheiden sich von den Verbindungen der Formel 5 (gemäss FR-OS 2 425 419) durch Vorliegen der *exo*-Konfiguration an C-2 und C-6 sowie von den Verbindungen der Formel 2 (gemäss DE-AS 1 218 643) durch Vorliegen einer Doppelbindung im tricyclischen Ringsystem. Die Verbindungen der Formel A sind somit neu.

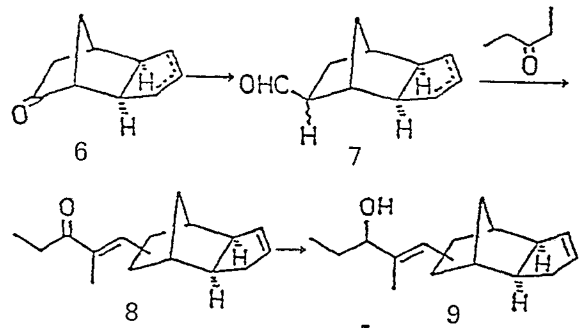

6 7

8 9

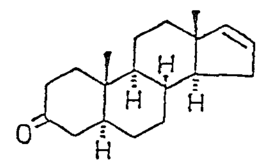

16-Androsten-3-on

Da die strukturverwandten Verbindungen der Formeln 2, 4 und 5 stets holzige Geruchsnoten mit Nebennoten in Richtung Iriswurzel oder Honig besitzen, war es überraschend, dass die Verbindungen der Formel A davon stark abweichende Geruchsnoten animalischen Typs aufweisen. Insbesonders das Keton 8, das durch Aldolkondensation des durch Glycidestersynthese aus dem bekannten Keton 6 erhaltenen Formyl-tricyclodecens 7 mit Diethylketon erhalten wurde, besitzt eine extrem intensive urinartige animalische Note, die in Intensität und Geruchstyp an das 16-Androsten-3-on erinnert. Durch Reduktion des Ketons 8 mit Natriumborhydrid oder Lithiumaluminiumhydrid wurde der Alkohol 9 mit animalisch-fettiger Note erhalten, die an Sandelholz erinnert.

Die Darstellung der Verbindungen der allgemeinen Formel A mit $R^1 = R^b = R^c = R^d$ = H erfolgt ausgehend von Dicyclopentadien (1), das bekannterweise unter saurer Katalyse hydratisiert wird. Hierbei wird das zunächst in der 2,6-endo-Form vorliegende Dicyclopentadien in die 2,6-exo-Form isomerisiert [G.L. Nelson und C.L. Kuo, Synthesis, 105 (1975), H.P. Kaufmann et al, „Fette, Seifen, Anstrichmittel", 67, 784 (1965)]. Das durch anschliessende Oxidation erhaltene Keton 6 [H.A. Bruson und Th.W. Riener, J. Amer. Chem. Soc., 67, 723 (1945)] wurde durch Glycidestersynthese in an sich bekannter Weise in den Aldehyd 7 überführt.

Eine andere Synthesemethode zur Darstellung des 2,6-exo-konfigurierten Aldehyds 7 beginnt mit endo-Dicyclopentadien (endo-1), das in Analogie zu bekannten Vorschriften [G.L. Nelson und CH.-L. Kuo, Synthesis, *1975*, 105] zum exo-Dicyclopentadien (exo-1) umgewandelt wurde. Selektive Hydroformylierung von exo-1 analog zur Darstellung von 3 [Y. Fujikura et al., Synth. Commun., 6, 199-207 (1976)] führte zu 7.

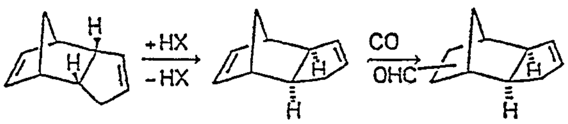


endo-1 exo-1 7

7

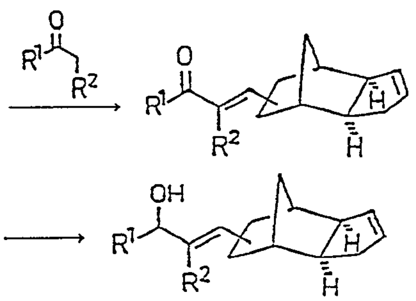

| | | |
|---|---|---|
| 10 | $R^1$=H,$R^2$=Me | 11 |
| 12 | $R^1$=H,$R^2$=Et | 13 |
| 14 | $R^1$=Me,$R^2$=H | 15 |
| 16a+ | $R^1$=Me,$R^2$=Me | 17a+ |
| 16b | $R^1$=Et,$R^2$=H | 17b |
| 8 | $R^1$=Et,$R^2$=Me | 9 |
| 18a+ | $R^1$=Me, $R_2$=n-Pent | 19a+ |
| 18b | $R^1$=n-Hex, $R^2$=Me | 19b |

Aldolkondensation von 7 mit aliphatischen Aldehyden (z.B. Propionaldehyd, Butyraldehyd) ergibt die ungesättigten Aldehyde 10, bzw, 12, die mit Lithiumaluminiumhydrid zu den ungesättigten Alkoholen 11 bzw. 13 reduziert werden. Die Aldehyde 10, 12 riechen frisch, holzig, süss und die Alkohole 11, 13 weich, holzig-buttrig mit Sandelholzaspekten. Durch Aldolkondensation mit Aceton, Methylethylketon, Diethylketon oder Methylhexylketon wurden die ungesättigten Ketone 14, 16a, b, 8 oder 18a, b (mit animalischen, urinartigen Tonalitäten erhalten, die zu den ungesättigten Alkoholen 15, 17a, b, 9 oder 19a, b (mit weichen, holzig-animalischen Noten) reduziert wurden.

Ausgehend von isomerisiertem dimerem Methylcyclopentadien (exo-20) können die höheren Homologen der Formel A [$R^a$, $R^b$, $R^c$ = H, $CH_3$ (2×H, 1×$CH_3$), $R^d$ = $CH_3$] auf analoge Weise dargestellt werden. Hydroformylierung des isomerisierten Dimethyldicyclopentadiens (Isomerengemisch) oder wahlweise Glycidestersynthese mit dem bekannten Keton 21 (EP-PS A1 0 039 232) ergeben den Aldehyd 22.

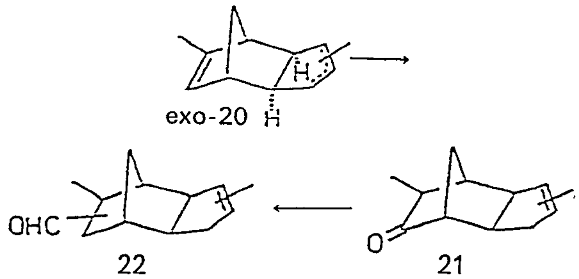

exo-20

22 21

22

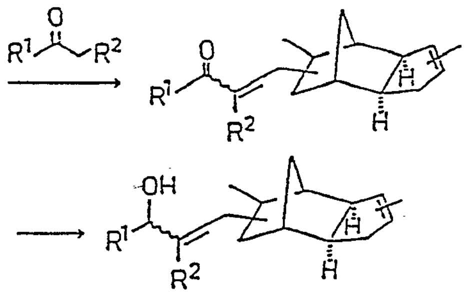

| | | |
|---|---|---|
| 23 | ($R^1$=H,$R^2$=Me) | 24 |
| 25 | ($R^1$=H,$R^2$=Et) | 26 |
| 27 | ($R^1$=Me,$R^2$=H) | 28 |
| 29 | ($R^1$=Et,$R^2$=Me) | 30 |
| 31a | ($R^1$=Me,$R^2$=n-Pent) | 32a |
| 31b | ($R^1$=n-Pent, $R^2$=Me) | 32b |

Aldolkondensation mit den entsprechenden aliphatischen Aldehyden oder Ketonen führen zu den Carbonylverbindungen 23, 25, 27, 29 und 31a, b (mit animalischen Geruchsnoten) und deren Reduktion mittels Natriumborhydrid oder Lithiumaluminiumhydrid zu den Alkoholen 24, 26, 28, 30 und 32a, b (mit sandelholzartigen Geruchsnoten). Bei der Sättigung des tricyclischen Systems durch vollständige katalytische Hydrierung geht die animalische Tonalität erheblich zurück.

Die selektive Hydrierung in der Seitenkette der α,β-ungesättigten Carbonylverbindungen 10, 12, 14, 16a, b, 8, 18a, b, 23, 25, 27, 29, 31a, b gelang durch Verwendung von Raney-Nickel und alkalischen Komponenten in Methanol. Die gesättigten Carbonylverbindungen 33, 35, 37, 39, 41 wurden durch Reduktion mit Natriumborhydrid in die entsprechenden Alkohole 34, 36, 38, 40, 42 überführt. Die Carbonylverbindungen 33, 35, 37, 39, 41 besitzen starke animalische Geruchsnoten mit wachsartig-fruchtigen Nebennoten. Die Alkohole 34, 36, 38, 40 und 42 riechen weich holzig-animalisch, in Richtung Sandelholz. Die aus dem Aldehyd 22 über die ungesättigten Carbonylverbindungen 23, 25, 27, 29, 31a, b auf gleiche Weise erhaltenen zugänglichen Dimethyl-Derivate 43-52 besitzen ähnliche Geruchseigenschaften bei etwas grösserer Fixierung.

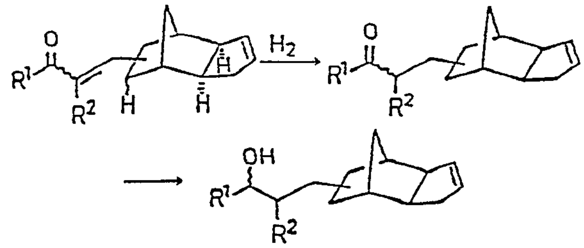

| | | | |
|---|---|---|---|
| 10 | 33 | ($R^1$=H,$R^2$=Me) | 34 |
| 12 | 35 | ($R^1$=H,$R^2$=Et) | 36 |
| 14 | 37 | ($R^1$=Me,$R^2$=H) | 38 |
| 16a | 39 | ($R^1$=Me,$R^2$=Me) | 40 |
| 8 | 41 | ($R^1$=Et,$R^2$=Me) | 42 |

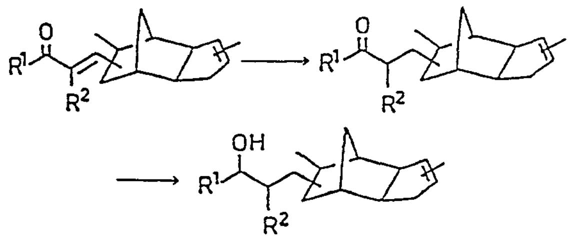

| | | | |
|---|---|---|---|
| 23 | 43 | $R^1$=H,$R^2$=Me | 44 |
| 25 | 45 | $R^1$=H,$R^2$=Et | 46 |
| 27 | 47 | $R^1$=Me,$R^2$=H | 48 |
| 29 | 49 | $R^1$=Me,$R^2$=Me | 50 |
| 31a,b | 51 | $R^1$=Et,$R^2$=Me | 52 |

*Physikalische Daten der oben beschriebenen Verbindungen sind in der folgenden Tabelle zusammengefasst*

| | $D_{4°}^{20°}$ | $n_D^{20°}$ | | $D_{4°}^{20°}$ | $n_D^{20°}$ |
|---|---|---|---|---|---|
| 10 | 1.0426 | 1.5382 | 33 | 1.0103 | 1.5045 |
| 11 | 1.0831 | 1.5291 | 34 | 1.0091 | 1.5022 |
| 14 | 1.0579 | 1.5322 | 37 | 1.0563 | 1.5298 |
| 15 | 1.0631 | 1.5291 | 38 | 1.0621 | 1.5200 |
| 16a,b | 1.0291 | 1.5287 | 39 | 1.0135 | 1.5205 |
| 17a,b | 1.0226 | 1.5214 | 40 | 0.9905 | 1.5173 |
| 8 | 1.0149 | 1.5252 | 41 | 0.9975 | 1.5096 |
| 9 | 0.9817 | 1.5050 | 42 | 0.9932 | 1.5105 |
| 18a,b | 0.9946 | 1.5120 | 43 | 1.0073 | 1.5105 |
| 19a,b | 0.9950 | 1.5159 | 44 | 1.0105 | 1.5076 |
| 23 | 0.9961 | 1.5131 | 47 | 0.9931 | 1.5013 |
| 24 | 0.9992 | 1.5096 | 48 | 0.9895 | 1.5075 |
| 27 | 1.0161 | 1.5213 | 51 | 0.9916 | 1.5006 |
| 28 | 1.0310 | 1.5192 | 52 | 0.9884 | 1.5062 |
| 29 | 1.0075 | 1.5195 | | | |
| 31a,b | 0.9932 | 1.5130 | | | |
| 32a,b | 0.9871 | 1.5032 | | | |

Die Herstellungsbeispiele 1-7 enthalten typische Reaktionsbedingungen, nach denen alle Verbindungen der allgemeinen Formel A hergestellt werden können.

Die Verbindungen der allgemeinen Formel A können aufgrund ihrer Geruchseigenschaften vorteilhaft als Riechstoffe verwendet werden. Die folgenden typischen Anwendungsbeispiele sollen die Erfindung erläutern, ohne sie einzuschränken.

*Beispiel 1*

*Darstellung von 8-Formyl-2,6-exo-tricyclo-[5.2.1.0$^{2,6}$]decen-4 (3)* (7)

Einer Lösung von 444 g (3 mol) 6, 550 g (4,5 mol) Chloressigsäureethylester und 1,5 g Phenothiazin in 300 ml Pyridin wurden bei −5 bis −10° C unter Rühren 270 g (5,0 mol) Natriummethylat portionsweise zugegeben. Anschliessend lässt man 300 ml Ether zulaufen. Nach 4 Stdn. Rühren bei −5° C werden 1,4 l 15%ige methanolische Natronlauge zugegeben. Man liess 10 Stdn. bei 0° C rühren, neutralisierte mit 1,5 l Essigsäure, verdünnte mit 2,0 l Wasser, extrahierte mehrfach mit Ether und arbeitete auf. Nach Einengen der vereinigten organischen Phasen verblieben 350 g Rohprodukt (dunkelgelbes Öl), das nach GC aus 70% 7 und 30% 6 bestand.

IR (Film): ν = 2700, 1720 cm$^{-1}$ (Aldehyd). $^1$H−NMR ($CCl_4$): δ = 5.1-5.8, m (olefin. H), 9.61 und 9.95 ppm, 2 „s" (−C<u>H</u>O). MS: m/z (%) = 162 (41, M$^+$), 144 (8), 133 (6), 129 (12), 118 (67), 105 (62), 96 (43), 95 (41), 91 (48), 77 (45), 67 (100).

$C_{11}H_{14}O$ (162.2)

*Beispiel 2*

*Darstellung von 2'-Methyl-3-oxo-pent-1'-enyl-2,6-exo-tricyclo[5.2.1.0$^{2,6}$]decen-4(3)* (8)

Einer Lösung von 178 g 7 (70%ig, Rohprodukt aus Beispiel 1) und 95 g Diethylketon in 400 ml Methanol wurden bei 0° C unter Rühren 40 ml Natronlauge (33%ig) innerhalb von ca. 15 min zugetropft. Man liess 3 Stdn. bei Raumtemperatur und 30 min bei Siedetemp. rühren. Nach Abkühlen wurden 100 g Essigsäure zugegeben. Man engte ein, nahm in ca. 300 ml Wasser auf, extrahierte mit Petrolether und arbeitete auf. Das Rohprodukt (200 g braunes Öl) wurde über eine 30 cm-Vigreux-Kolonne destilliert. Man erhielt 105 g (60%) 8 als farbloses Öl [Kp (0.4 mbar) = 120° C] mit starkem urinartigem Geruch.

IR (Film): 1680, 1640 cm$^{-1}$ (α,β-unges. Keton). $^1$H−NMR: Abb. 1, MS: Abb. 2.

$C_{16}H_{22}O$ (230.4).

*Beispiel 3*

*Darstellung von 2'-Methyl-3'-hydroxy-pent-1'-enyl-2,6-exo-tricyclo[5.2.1.0$^{2,6}$]decen-4(3)* (9)

Zu einer Lösung von 23 g (0,1 mol) des Ketons 8 in 15 ml Ethanol wurde innerhalb von 20 min eine Lösung von 5 g Natriumborhydrid und 0,1 g Natriumhydroxid in 10 ml Wasser hinzugetropft. Nach 3 Stdn. Rühren bei Raumtemp. gab man 200 ml Wasser zu und extrahierte mit Ether. Die vereinigten organischen Phasen wurden neutralgewaschen, eingeengt und über eine 30 cm-Vigreux-Kolonne destilliert. Man erhielt 15 g (65%) 9 als farbloses Öl [Kp (0.5 mbar) = 118-120° C] mit buttrigem, sandelholzartigem Geruch.

IR (Film): 3380 cm$^{-1}$ (OH). $^1$H−NMR ($CCl_4$): δ = 0.78, t, J = 7 Hz ($-CH_2-CH_3$), 1.60, br.s (2'-$CH_3$), 3.75, br.t, J = 6 Hz (−C<u>H</u>(OH)−), 5.15, m (olefin. 1'-H), 5.25-5.75 ppm, m (olefin. 3-, 4-, 5-H). MS: m/z (%) = 232 (3, M$^+$), 214 (2), 203 (79), 185 (16), 175 (9), 149 (66), 147 (36), ..., 99 (94), 91 (67), 79 (70), 67 (100).

$C_{16}H_{24}O$ (232.4).

*Beispiel 4*

*Darstellung von 2'-Formyl-prop-1'-enyl-2,6-exo-tricyclo[5.2.1.0$^{2,6}$]decen-5(3)* (10)

Man gab einer Lösung von 162 g 7 (70%ig, Rohprodukt nach Beispiel 1) in 200 ml Methanol

bei ca. 10° C 6,0 g Natriummethylat zu. Unter Rühren liess man innerhalb von 1 Stde. 64 g (1,1 mol) Propionaldehyd zutropfen. Nach 5 min Rühren bei Siedetemp. und anschliessendem Abkühlen neutralisierte man mit Essigsäure. Nach üblicher Aufarbeitung wurde das erhaltene Rohprodukt (220 g) über eine 15 cm-Vigreux-Kolonne destilliert. Man erhielt 132 g (65%) 10 als farbloses Öl; Kp (1 mbar) = 108-110° C.

IR (Film): 2702, 1685, 1635 $cm^{-1}$ (α,β-unges. Aldehyd), $^1H$-NMR: δ = 1.73, d, J = 2.5 Hz (olef. Methyl), 5.3-5.8, m (olefin 3-, 4-, 5-H), 6.18 und 6.33, 2m (olefin. 1-H, 9-exo-/9-endo-), 927 ppm (-CHO). MS: m/z (%) = 202 (37, $M^+$), 187 (3), 173 (9), 136 (35), 135 (74), 131 (13), 117 (34), 106 (86), 105 (82), 97 (51), 95 (53), 91 (100).

$C_{14}H_{18}O$ (202.3).

*Beispiel 5*

*Darstellung von 3'-Hydroxy-2'-methyl-prop-1'-enyl-2,6-exo-tricyclo[5.2.1.0$^{2,6}$]decen-4(3)* (11)

Unter Rühren wurde bei Raumtemp. eine Lösung von 34 g (0,2 mol) 10 in 50 ml Ether zu einer Suspension von 2.5 (0.07 mol) Lithiumaluminiumhydrid in 120 ml Ether hinzugetropft. Nach 3 Stdn. Rühren bei Siedetemp. gab man erst 5 ml Ethylacetat und dann 10 ml Wasser hinzu und arbeitete auf. Destillation über eine 15 cm-Vigreux-Kolonne ergab 29.5 g (87%) 11 als farbloses Öl; Kp (1 mm) = 123° C.

IR (Film): 3400 $cm^{-1}$ (OH). NMR ($CCl_4$): δ = 1.63, d, J = 2 Hz (olefin. $CH_3$), 3.79, br.s ($-CH_2-OH$), 5.12, m (olefin. 1'-H), 5.2-5.7 ppm (olefin. 3-, 4-, 5-H). MS: m/z (%) = 204 (5, $M^+$), 189 (4), 186 (2), 173 (18), 149 (100), 137 (12), 131 (16), 119 (25), 107 (27), 105 (30), 91 (62), 79 (83), 67 (76).

$C_{14}H_{20}O$ (204.3).

*Beispiel 6*

*2'-Methyl-3'-oxo-butyl-2,6-exo-tricyclo-[5.2.1.0$^{2,6}$]decen-4(3)* (41) *durch selektive Hydrierung*

Eine Lösung von 43.2 g (0.2 mol) 16a (analog Beispiel 2) und 0.4 g Natriumhydroxid in 240 ml Methanol wurde mit 0.8 g Raney-Nickel versetzt und 7 Stdn. in einer Wasserstoff-Atmosphäre geschüttelt (Raumtemperatur/Normaldruck); es wurden 4.44 l Wasserstoff aufgenommen (theoretische Aufnahme 4.48 l). Filtration, Einengen und Aufarbeiten ergaben 44 g Rohprodukt, das über eine 1 m-Drehbandkolonne destilliert wurde. Man erhielt 34.5 g farbloses Öl, KP (0.5 mbar) = 86° C, das nach GC zu ca. 70% aus dem selektiv hydrierten Keton 39 und zu ca. 30% aus dem entsprechenden vollständig hydrierten Keton bestand. 39: IR (Film): ν = 1702 $cm^{-1}$ (gesättigtes Keton). $^1H$-NMR ($CCl_4$): δ = 1.03, d, J = 7 Hz (2'-$CH_3$), 2.02, p ($CH_3$-4'), 5.30-5.65 ppm, m (CH-3.4). MS: m/z (%) = 218 (10, $M^+$), 200 (3), 175 (5), 151 (46), 147 (100), 146 (18), 133 (20), 131 (16), 117 (8), 109 (11), 105 (12), 91 (25), 85 (7), 81 (32), 80 (30), 79 (75), 67 (78), 66 (78), ..., 43 (36).

$C_{15}H_{22}O$ (218.2).

*Beispiel 7*

*Darstellung von 2'-Methyl-3'-hydroxy-butyl-2,6-exo-tricyclo[5.2.1.0$^{2,6}$]decen-4 (3)* (40)

Zu einer Aufschlämmung von 2.7 g (0.07 mol) Lithiumaluminiumhydroxid in 100 ml Diethylether wurde unter Rühren eine Lösung von 30 g (0.136 mol) 39 (nach Beispiel 6) in 150 ml Diethylether zugetropft. Nach 2 Stdn. Rühren bei Siedetemperatur wurde vorsichtig mit Ethylacetat versetzt und aufgearbeitet. Das Rohprodukt wurde über eine 20 cm-Vigreux-Kolonne destilliert. Man erhielt 24 g (78%) 40 als farbloses Öl, Kp (2 mbar) = 130-133° C.

IR (Film): ν = 3350 $cm^{-1}$ (Hydroxyl-). $^1H$-NMR: δ = 0.87 und 0.93 ($CH_3$-4'), 1.04, d, J = 7 Hz (2'-$CH_3$), 3.3-3.6, m (CH-3'), 5.2-5.6 ppm, m (CH-3.4). MS: m/z (%) = 220 (8, $M^+$), 153 (32), 135 (53), 131 (15), 117 (11), 107 (27), 105 (13), 93 (33), 91 (31), 81 (22), 80 (44), 79 (56), ..., 67 (87), 66 (100).

$C_{15}H_{24}O$ (220.3).

*Beispiel 8*

*Parfümöl mit blumig-aldehydischer Note*

| | |
|---|---|
| α-Hexylzimtaldehyd | 200 |
| Phenylethylalkohol | 140 |
| Acetylcedren | 100 |
| Vetiverylacetat | 80 |
| Hydroxycitronellal | 80 |
| γ-Methyljonon | 80 |
| Citronellol | 60 |
| Benzylacetat | 50 |
| Geraniol | 50 |
| Bergamott-Öl, Reggio | 50 |
| Trichlormethylphenylcarbinylacetat | 20 |
| Coumarin | 20 |
| Moschus Ambrette | 20 |
| Moschus Keton | 20 |
| Eichenmoos-Extrakt | 20 |
| Cyclopentadecanolid | 9 |
| | 999 |

Dieses Parfümöl besitzt einen ausgewogenen blumig-aldehydischen Duftkomplex mit herbem, holzigem Fond.

a) Bei Zugabe von 1 Teil einer 10%igen Lösung des Ketons 8 oder des Ketons 29 in Dipropylenglykol erhält das Parfümöl einen sehr gewünschten animalischen Aspekt, der an Zibeth oder Tonkin-Moschus erinnert.

b) Bei Zugabe von 1 Teil des Aldehyds 10 oder des Aldehyds 23 wird der holzig-animalische Fond betont.

*Beispiel 9*

*Parfümbase vom Sandelholztyp*

| | |
|---|---|
| Cedren | 80 |
| Acetylcedren | 350 |
| Verbindung 9 | 350 |
| Amyrisöl | 200 |
| Verbindung 8 (1%ig in Dipropylenglykol) | 20 |
| | 1000 |

Diese Parfümbase besitzt einen ausgeprägten animalisch-holzigen Duft vom Sandelholztyp.

*Beispiel 10*

*Parfümöl mit Maiglöckchen-Duft*

| | |
|---|---|
| Hydroxycitronellal | 200 |
| Phenylethylalkohol | 200 |
| α-Hexylzimtaldehyd | 200 |
| Linalool | 100 |
| Citronellol | 80 |
| Hydroxyisohexyltetrahydrobenzaldehyd | 30 |
| Linalylacetat | 25 |
| Rosenholzöl | 25 |
| Geraniol | 20 |
| Ylang-Ylang-Öl | 15 |
| Benzylacetat | 10 |
| Phenylacetaldehyd-dimethylacetat | 10 |
| Zimtöl, 10%ig in Diethyl-phthalat | 10 |
| Indol, 10%ig in Diethyl-phthalat | 10 |
| Heptanal, 10%ig in Diethyl-phthalat | 5 |
| | 940 |

Ein Zusatz von jeweils 60 Teilen der Verbindungen 11 bzw. 24 verleiht dem Parfümöl mit Maiglöckchenduft eine weiche holzige Note und verbesserte Fixierung.

*Beispiel 11*

*Parfümöl mit balsamisch-süsser Note*

| | |
|---|---|
| Phenylethylalkohol | 180 |
| Patchouliöl, Singapore | 120 |
| Bergamottöl | 90 |
| Hydroxycitronellal | 70 |
| γ-Methyljonon | 60 |
| Moschus Keton | 60 |
| Moschus Ambrette | 50 |
| α-Hexylzimtaldehyd | 50 |
| Eugenol | 40 |
| Lavendelöl, französisch | 40 |
| Ethylvanillin, 10%ig in Dipropylenglykol | 30 |
| Benzoe-Resinoid, Siam | 25 |
| Phenylethylacetat | 25 |
| Benzylacetat | 25 |
| Coumarin | 25 |
| Geraniumöl, Bourbon | 25 |
| Perubalsamöl | 20 |
| Ethylenbrassylat | 15 |
| Isoeugenol | 10 |
| Kamillenöl | 10 |
| | 970 |

Bei Zugabe von 30 Teilen des Keton-Gemisches 18a + 18b bzw. 31a + 31b wurde eine Betonung der dunkel-balsamischen Note unter gleichzeitiger Abrundung erzielt.

## Patentansprüche

1. 2,6-Exo-konfigurierte Tricyclo[5.2.1.$0^{2,6}$]-decan-Derivate der allgemeinen Formel A, worin

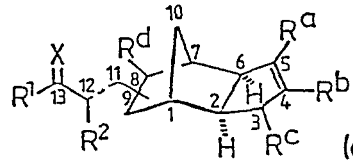

A

(d,l)

$R^a=R^b=R^c=R^d=H$

$R^a,R^b,R^c=H,CH_3$ ($2\times H, 1\times CH_3$), $R^d=CH_3$

X: =O, $<^{OH}_{H}$

$R^1,R^2$: H, $C_1$-$C_6$-Alkyl

– $R^a$, $R^b$, $R^c$, $R^d$ Wasserstoffreste oder $R^d$ eine Methylgruppe und $R^a$, $R^b$, $R^c$ Wasserstoff- oder Methylreste bedeuten, wobei einer der Substituenten ein Methylrest und die beiden übrigen Wasserstoffreste bedeuten,

– die gestrichelte Linie zwischen C-11/C-12 eine C-C-Doppelbindung oder eine C-C-Einfachbindung bedeutet,

– $R^1$ und $R^2$ Wasserstoff- oder geradkettige oder verzweigte $C_1$-$C_6$ Niederalkylreste bedeuten, und

– X eine Carbonyl- oder Hydroxylfunktion darstellt.

2. Verfahren zur Herstellung der Tricyclodecanderivate der Formel A aus Formyl-2,6-exotricyclodecan-Derivaten, die aus Dicyclopentadien oder dimerem Methylcyclopentadien in an sich bekannter Weise entweder durch saure Hydratisierung, anschliessende Oxidation und Glycidester-Synthese oder durch Säure-induzierte Isomerisierung und anschliessende Hydroformylierung erhalten werden, dadurch gekennzeichnet, dass man diese Formylderivate durch Aldolkondensation mit aliphatischen Aldehyden oder Ketonen wahlweiser selektiver Hydrierung der Seitenkette und/oder Reduktion der Carbonyl- zur Hydroxylgruppe in die Verbindungen der Formel A überführt.

3. Verwendung der Verbindungen nach Anspruch 1 als Riechstoffe oder Bestandteile von Riechstoff-Mischungen bzw. Parfümölen für kosmetische oder technische Parfümierungen.

## Claims

1. 2,6-Exo-configured tricyclo[5.2.1.$0^{2,6}$]decane derivatives of the general formula A, wherein

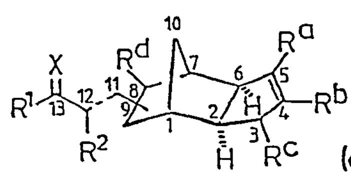

A

(d,l)

$R^a=R^b=R^c=R^d=H$

$R^a,R^b,R^c=H,CH_3$ ($2\times H, 1\times CH_3$), $R^d=CH_3$

X: =O, $<^{OH}_{H}$

$R^1,R^2$: H, $C_1$-$C_6$-Alkyl

– $R^a$, $R^b$, $R^c$, $R^d$ are hydrogen or $R^d$ is a methyl group and $R^a$, $R^b$, $R^c$ are hydrogen or methyl groups, one of these substituents being a methyl group and both the others being hydrogen,
– the broken line between C11/C12 represents a C-C double bond, or C-C single bond,
– $R^1$ and $R^2$ are hydrogen or straight-chain or branched-chain $C_1$-$C_6$ lower alkyl groups and
– X represents a carbonyl- or hydroxyl function.

2. Method for the preparation of tricyclodecane derivatives of the formula A starting from formyl-2,6-exo-tricyclo-decane derivatives which, in a manner known *per se*, are obtained from dicyclopentadiene or dimeric methylcyclopentadiene by acid hydration, followed by oxidation and glycide ester synthesis, or by acid-induced isomerization, followed by hydroformylization, characterized in that said formyl derivatives are converted into compounds of the formula A by aldol condensation with aliphatic aldehydes or ketones taking place under selective hydrogenation of the side chain and/or reduction of the carbonyl to the hydroxyl group.

3. Use of compounds according to claim 1 as scent materials or ingredients of scent mixtures or perfume oils for cosmetic or technical perfuming.

## Revendications

1. Dérivés de Tricyclo[5.2.1.$0^{2,6}$]décane à configuration 2,6-exo de formule générale A, dans laquelle

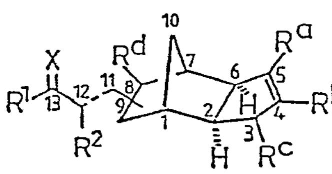

A

(d,l)

$R^a = R^b = R^c = R^d = H$

$R^a, R^b, R^c = H, CH_3$ ($2 \times H, 1 \times CH_3$), $R^d = CH_3$

X: = O <OH H

$R^1, R^2$: H, $C_1$-$C_6$–Alkyl

– $R^a$, $R^b$, $R^c$, $R^d$ représentent chacun un hydrogène ou $R^d$ représente un groupe méthyle et $R^a$, $R^b$, $R^c$ représentent un hydrogène ou un groupe méthyle et où un des substituants est un groupe méthyle et les deux groupes restants sont chacun un hydrogène,
– la ligne discontinue entre C-11/C-12 représente une double liaison C-C- ou unique liaison C-C,
– $R^1$ et $R^2$ représentent chacun un hydrogène ou un groupe alkyle inférieur, droit ou ramifié, en $C_1$-$C_6$, et
– X représente une fonction carbonyle ou hydroxyle.

2. Procédé de fabrication des dérivés de Tricyclodécane de formule A à partir des dérivés Formyl-2,6-exotricyclodécane qui sont obtenus d'une manière connue en soi à partir du dicyclopentadiène ou du dimère de Méthylcyclopentadiène soit par une hydratation acide suivie d'une oxydation et d'une synthèse-esterglycidique ou par une isomérisation induite par un acide suivie par une hydroformylation, caractérisé en ce que l'on transforme ces dérivés Formyl par condensation aldolique avec des aldéhydes aliphatiques ou des cétones, hydrogénation sélective facultative de la chaîne latérale et/ou réduction du groupe carbonyle en groupe hydroxyle, en composés de formule A.

3. Utilisation des composés selon la revendication 1 comme parfum ou constituants de mélanges de parfums ou d'essences de parfums pour parfumages cosmétiques ou techniques.